# EUROPÄISCHE PATENTSCHRIFT

(11) **EP 1 886 652 B1**
(45) Veröffentlichungstag und Bekanntmachung des Hinweises auf die Patenterteilung: **22.02.2012**
(21) Anmeldenummer: 07014044.7
(22) Anmeldetag: 18.07.2007
(51) Int. Cl.: A61F 7/00, A61B 18/02, A61N 5/06

(54) **Vorrichtung zur Benutzung in der Kryotherapie**
Device for use in cryotherapy
Dispositif destiné à l'utilisation en cryothérapie

(30) Priorität: 11.08.2006 DE 102006037670
(43) Veröffentlichungstag der Anmeldung: 13.02.2008
(73) Patentinhaber: Damm, Hans, 76473 Iffezheim (DE)
(72) Erfinder:
(74) Vertreter: Lemcke, Brommer & Partner

(56) Entgegenhaltungen:
- WO-A-99/27863
- WO-A1-00/44297
- WO-A2-2006/063334
- GB-A- 2 291 499
- US-A- 5 820 626
- US-A- 5 976 123
- US-A- 5 997 530
- US-A1- 2004 147 986
- US-A1- 2006 153 273

## Beschreibung

Die Erfindung betrifft eine Vorrichtung zur Benutzung in der Kryotherapie mit einer Austrittsöffnung, durch die Kältemittel, insbesondere Gas, auf eine zu behandeinde Stelle geleitet wird.

Unter der Kryotherapie versteht man die Behandlung von verschiedenen Leiden durch oberflächliche Einwirkung großer Kälte. Diese führt bekanntermaßen zu verschiedenen physiologischen Wirkungen, z.B. der Herabsetzung der Schmerzschwelle, einer Verminderung der Spastik, einer Entspannung von Muskelfasern oder auch einer blutstillenden Wirkung.

Früher wurde hierzu insbesondere verflüssigter Stickstoff benutzt, der allerdings aufgrund der speziellen Bedingungen, unter denen er gelagert werden muss, schwere unhandliche Vorrichtungen benötigt. Aus diesen Vorrichtungen verdampfte der verflüssigte Stickstoff ständig, was ungewünschte Verluste verursacht. Außerdem ist flüssiger Stickstoff nur mit hohem Aufwand zu transportieren und großflächig bereitzustellen, was seinem Einsatz entsprechend entgegensteht.

Es ist diesbezüglich beispielsweise aus der EP-0 633 008 bekannt, statt Stickstoff verflüssigtes Kohlendioxyd zu verwenden, das aus einer Druckflasche entnommen wird und über eine druckfeste Leitung zu einer pistolenartigen Vorrichtung geführt wird, in der es entspannt und dann ausgestoßen wird. Dabei kann das ausgestoßene Gas Temperaturen von - 75 °C erreichen, wodurch die hiermit behandelte Haut innerhalb weniger Sekunden von ihrer Normaltemperatur (ca. 32 °C) heruntergekühlt werden kann auf Temperaturen von 0 bis 5 °C.

Es hat sich dabei herausgestellt, dass der entsprechende Thermoschock z. B. zu einer neuroreflektorischen Ausdehnung der Gefäße im betroffenen Bereich führt, wodurch Entzündungsmediatoren schneller abtransportiert werden.

Weitere Wirkungsweisen sind noch nicht abschließend wissenschaftlich erforscht.

Mit der Zufuhr von Energie in tiefere Gewebebereiche beispielsweise mittels Laser oder starken Lichtblitzen, wie dies z.B. in der US-A-5976123 oder der US-2004/0147986 A1 vorgeschlagen wird, sind diese Wirkungsweisen jedoch nicht vergleichbar.

Aufgabe der vorliegenden Erfindung ist es nun, eine Vorrichtung der beschriebenen Art dahingehend zu verbessern, dass mit ihr eine noch bessere Wirkung in dem mit ihr behandelten Organismus erreicht werden kann.

Diese Aufgabe wird mit einer Vorrichtung nach Anspruch 1 dadurch gelöst, dass die oben beschriebene Vorrichtung weiterhin mit einer Quelle für Licht, das auf die mit Kältemittel behandelte Stelle gerichtet ist, versehen ist.

Unter Licht wird dabei im Rahmen der hier vorliegenden Erfindung im wesentlichen "sichtbares Licht" verstanden.

Der Erfindung liegt dabei die Erkenntnis zugrunde, dass durch eine lokale Kombination von Kälte und Licht im Organismus eine verbesserte Wirkung der oben beschriebenen Art zu erreichen ist. Insbesondere wird hierbei das Licht auf den Bereich fokussiert, in dem das Kältemittel auf die Haut eines Patienten auftrifft.

Eine besonders gute Wirkung hat ein Licht mit blauer Farbe, insbesondere bei einer Wellenlänge von ca. 470 nm. Durch dieses wird im Bereich der Behandlung außerdem der Sauerstoffaustausch im Gewebe verbessert und somit die gewünschten therapeutischen Ergebnisse verstärkt.

Bei einer bevorzugten Ausführungsform ist es dabei außerdem möglich, dass das Licht in seiner Farbe veränderlich ist. Sie kann somit auf unterschiedliche Hauttypen Schmerztiefen oder ähnliche Faktoren die auf spezielles Licht positiv reagieren, vom Therapeuten individuell eingestellt werden.

Die Vorrichtung wie oben bescrieben wird zur Benutzung in der Kryotherapie mit einer Messeinrichtung zur Ermittlung der Temperatur der Haut eines Patienten versehen. Diese Temperaturmessung erfolgt insbesondere über eine kontaktiose Infrarot-Messung.

Um dabei über den gesamten Bereich, der von Haut bei Behandlung in der Kryotherapie durchlaufen wird und etwa zwischen 35 und 0 °C liegt, ein reproduzierbares Messergebnis zu erhalten, wird vorgeschlagen, die Infrarotmessung über einen Thermopile-Sensor vorzunehmen. Dieser ist mit einer Linse versehen, so dass er bei einem Abstand von etwa 5 bis 20 cm zu der Stelle, an der er die Temperatur messen soll, in einem relativ begrenzten Kreis mit einem Durchmesser von ca. 5 bis 15 mm misst.

Es ist somit gewährleistet, dass tatsächlich in dem Bereich gemessen wird, in dem die Kryotherapie lokal begrenzt eingesetzt wird.

Bei einer bevorzugten Ausführungsform wird der verwandte Thermopile-Sensor in einer als Wärmesenke fungierenden Hülle eingebaut, die zur Kompensation der Einflüsse der Umgebungstemperatur benutzt wird.

Der entsprechende Sensor ist mit einer Auswertelogik, die durch das Kältemittel selbst verursachte Messergebnisse selektiv kompensiert, verbunden, so dass hierdurch keine Verfälschung der an der Haut existierenden zu messenden Temperatur erfolgt.

Weitere Vorteile und Merkmale der Erfindung ergeben sich aus der nachfolgenden Beschreibung eines Ausführungsbeispiels. Dabei zeigt.

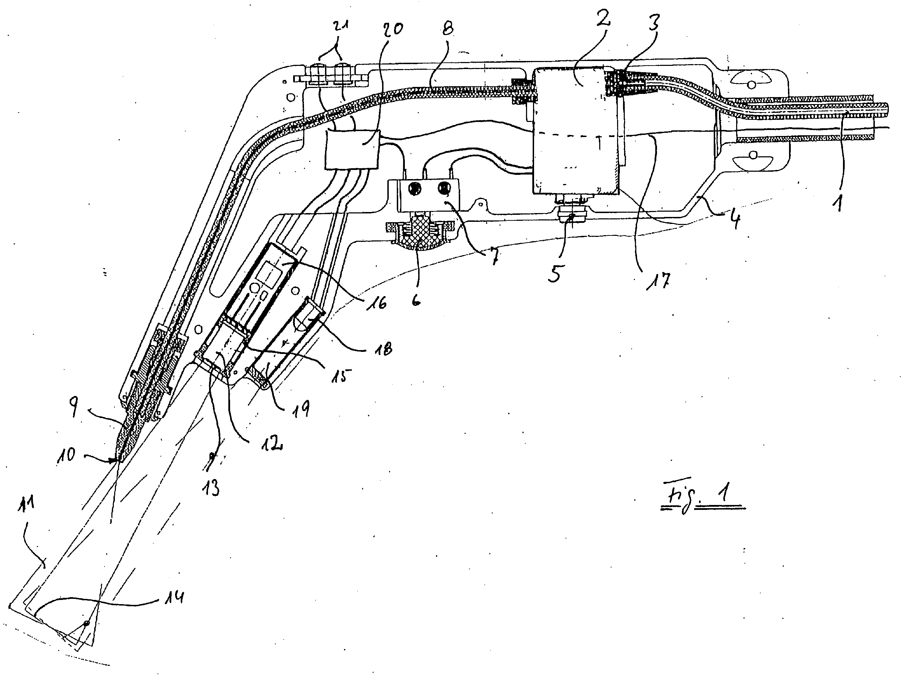

- Figur 1: die Schnittansicht einer erfindungsgemäßen Vorrichtung.

In Figur 1 ist die Schnittansicht einer "Pistole" zu erkennen, die in der Kryotherapie verwandt wird.

Dabei wird über einen Druckschlauch 1 unter Druck befindliches flüssiges Kohlendioxyd zu einem Ventil 2 geführt. An dieses Ventil 2 ist der Druckschlauch 1 mit einer entsprechenden Anschlussverbindung 3 angeschraubt.

Das Ventil 2 ist innerhalb eines Gehäuses 4 angebracht und im hier dargestellten Ausführungsbeispiel mit einer von außerhalb des Gehäuses zugänglichen Dosierschraube 5 versehen, mit der eine maximale Öffnungsstellung des Ventils einstellbar ist.

Das Ventil wird dabei über einen von außen durch einen Druckknopf 6 zu betätigenden Mikroschalter 7 betätigt.

Durch das Drücken des Druckknopfes 6 wird der Mikroschalter 7 geschlossen und damit das Ventil 2 elektromagnetisch geöffnet. Dadurch kann das im Druckschlauch 1 befindliche flüssige Kohlendioxyd in das Ventil 2 hineinströmen. Das flüssige Kohlendioxyd fließt dann durch eine Leitung 8 zu einer Düse 9, die an ihrem vorderen Ende mit einer Austrittsöffnung 10 versehen ist. Nach dieser Düse entspannt sich das flüssige Kohlendioxyd zu Kohlendioxydgas, tritt in einem im wesentlichen kegelförmigen Strahl 11 aus und trifft auf eine (hier nicht dargestellte) zu behandelnde Stelle eines Patienten. Dort bewirkt das kalte Kohlendioxyd die mit der Kryotherapie verfolgten Effekte.

Hierbei wirkt insbesondere die lokale starke Abkühlung. Diese wird bei dem hier dargestellten Ausführungsbeispiel über eine kontaktlose Infrarot-Messung verfolgt, wozu ein Thermopile-Sensor 12 vorgesehen ist. Dieser Thermopile-Sensor weist an seinem vorderen Ende eine Linse 13 auf, so dass ein relativ enger Bereich durch den Thermopile-Sensor 12 überwacht wird. Insbesondere ist diese Linse so gewählt, dass in einem Abstand von etwa 5 bis 20 cm ein Messbereich 14 erfasst wird, der direkt an der mit Kältemittel zu behandelnden Stelle eines Patienten liegt und einen Durchmesser von etwa 5 bis 15 mm hat.

Der Sensor selbst ist in einer als Wärmesenke fungierenden Hülse 15 integriert, durch die Umgebungstemperatureinflüsse ausgeglichen werden.

Des weiteren ist dem Thermopile-Sensor eine Auswertelogik 16 zugeordnet, in der direkt durch das Kältemittel, im hier vorliegenden Fall also Kohlendioxyd erzeugte Temperaturen ausgeblendet werden, um lediglich die tatsächliche Hauttemperatur ermitteln zu können.

Die von der Auswertelogik 16 ermittelte Temperatur wird dann über eine Leitung 17 an eine zentrales Anzeigeeinheit übermittelt, wo der Bediener einer entsprechenden Vorrichtung die Temperatur ablesen kann.

Bei der hier dargestellten Vorrichtung ist außerdem noch eine Lichtquelle 18 vorgesehen, mit der die mit Kältemittel zu behandelnde Stelle eines Patienten mit Licht bestrahlt werden kann. Das Licht wird hierzu über unterschiedlichste Mittel, z.B. Linsen oder Lichtkanäle 19 fokussiert, so dass es eine ausreichende Intensität hat.

Des weiteren wird für die Farbe der Lichtquelle insbesondere Blau gewählt, was Licht mit einer Wellenlänge von etwa 470 nm zur Folge hat. Hierdurch wird eine besondere Tiefenwirkung mit einem besonders guten Sauerstoffaustausch im Gewebe erzielt.

Dabei kann die Lichtquelle 18 aber durch eine entsprechende Steuerlogik 20 auch auf andere Farben eingestellt werden.

Durch die Steuerlogik 20 können auch durch einen Bediener leicht abzulesende Signallampen 21 angesteuert werden, die für die unterschiedlichsten Zwecke bedarfsgerecht einzusetzen sind.

Die vorliegende Einrichtung ermöglicht somit eine erhebliche Verbesserung der kryotherapeutischen Erfolge.

## Patentansprüche

1. Vorrichtung zur Benutzung in der Kryotherapie mit einer Austrittsöffnung (10), durch die Kältemittel, insbesondere Gas, auf eine zu behandelnde Stelle geleitet wird,
und mit
einer Quelle (18) für Licht, das auf die mit Kältemittel behandelte Stelle gerichtet ist, wobei
das Licht fokussiert ist und
eine blaue Farbe hat, insbesondere bei einer Wellenlänge von ca. 470 nm,
wobei eine Messeinrichtung für die Temperatur der behandelten Stelle vorhanden ist, und
die Temperaturmessung über eine kontaktlose Infrarot-Messung mittels
einem Thermopile-Sensor erfolgt, der mit einer Linse versehen ist zur Temperaturmessung in einem Kreis von ca. 5 bis 15 mm bei einem Abstand von ca. 5 bis 20 cm,
wobei dem Sensor (12) eine Auswertelogik (16) nachgeschaltet ist, die durch das Kältemittel selbst verursachte Messergebnisse selektiv kompensiert.

2. Vorrichtung gemäß Anspruch 1,
wobei
der Sensor (12) in einer als Wärmesenke fungierenden Hülse (15) sitzt zur Kompensation der Umgebungswärme.

3. Vorrichtung gemäß Anspruch 1,
wobei sie eine Steuerlogik (20) aufweist, durch die Signallampen (21) angesteuert werden, die durch einen Bediener abzulesen sind.

## Claims

1. Device for use in cryotherapy, having an outlet opening (10) through which coolant, especially gas, is guided to a location to be treated, and having a source (18) for light which is directed onto the location treated with coolant, the light being focussed and having a blue colour, especially at a wavelength of about 470 nm, wherein a measuring device for the temperature of the treated location is provided, and the temperature measurement is effected by means of a contactless infrared measurement by means of a thermopile sensor, which is provided with a lens, for temperature measurement in a circle of about from 5 to 15 mm at a distance of about from 5 to 20 cm, there being arranged downstream of the sensor (12) an evaluating logic device (16) which selectively compensates for measurement results caused by the coolant itself.

2. Device according to claim 1,
wherein the sensor (12) is seated in a sleeve (15) which acts as heat sink to compensate for the ambient heat.

3. Device according to claim 1,
wherein the device has a control logic device (20) by means of which signal lamps (21) are actuated, which lamps are to be read by an operator.

## Revendications

1. Dispositif destiné à être utilisé dans la cryothérapie, avec une ouverture de sortie (10) à travers laquelle le fluide réfrigérant, en particulier un gaz, est conduit vers un endroit à traiter,
et avec une source (18) de lumière qui est dirigée sur l'endroit traité avec le fluide réfrigérant,
dans lequel
la lumière est focalisée et a une couleur bleue, en particulier à une longueur d'onde d'environ 470 nm,
un dispositif de mesure de la température de l'endroit traité est prévu et
la mesure de température est réalisée par une mesure infrarouge sans contact au moyen d'un capteur à thermopile qui est pourvu d'une lentille pour la mesure de température dans un cercle d'environ 5 à 15 mm à une distance d'environ 5 à 20 cm,
une logique d'évaluation (16) qui compense sélectivement les résultats de mesure provoqués par le fluide réfrigérant lui-même est placée en aval du capteur (12).

2. Dispositif selon la revendication 1,
dans lequel
le capteur (12) est logé dans un manchon (15) faisant office de dissipateur thermique pour compenser la chaleur ambiante.

3. Dispositif selon la revendication 1,
lequel présente une logique de commande (20) qui commande des lampes de signalisation, (21) qui peuvent être lues par un opérateur.
